(19) Europäisches Patentamt
European Patent Office
Office européen des brevets

(11) EP 0 793 952 A1

(12) EUROPEAN PATENT APPLICATION

(43) Date of publication:
10.09.1997 Bulletin 1997/37

(51) Int. Cl.⁶: A61F 13/15

(21) Application number: 96830097.0

(22) Date of filing: 07.03.1996

(84) Designated Contracting States:
AT BE CH DE DK ES FI FR GB GR IE IT LI LU NL PT SE

(71) Applicant: THE PROCTER & GAMBLE COMPANY
Cincinnati, Ohio 45202 (US)

(72) Inventor: Bewick-Sonntag, Christopher Philip
65125 Pescara (IT)

(74) Representative: Hirsch, Uwe Thomas et al
Procter & Gamble European Service GmbH,
Sulzbacher Strasse 40-50
65824 Schwalbach am Taunus (DE)

(54) **Absorbent articles having fluid contact angle gradients between the topsheet and absorbent core**

(57) The present invention relates to an absorbent article comprising a liquid pervious topsheet, an absorbent core and a breathable backsheet. The absorbent article has a fluid contact angle gradient between the topsheet and absorbent core and exhibits a reduced tendency to adhere to the skin of the wearer.

**Description**

Technical field of the Invention

The present invention relates to disposable absorbent articles such as diapers, sanitary napkins and incontinence pads which exhibit reduced adhesion of the product to skin of the user.

Background of the Invention

The primary consumer needs which underlie development in the absorbent article field, in particular catamenials is the provision of a product delivering both a high protection and comfort level.

One highly desirable means of providing improved comfort has been through the development of absorbent articles, which more readily conform to the contours of the body, for example by the utilisation of thinner products. The disadvantage of such products is that as a result of the increased contact between the wearer and the absorbent article and reduced product to skin gapping, the air residence time within the product itself and in-between the product and the body of the wearer increases. Consequently, the exchange of air within the product is less frequent. As a result the temperature of the skin of the wearer increases, causing perspiration and discomfort, a problem which is commonly referred to as stickiness. Under stressed wearing conditions, such as in hot and humid environments, during extended periods of use or during physical excertion, the stickiness problem will ultimately result in at least the partial adhesion of the wearer facing surface of the absorbent article to the skin of the wearer.

Another means of addressing consumer needs in respect of comfort is the use of particular topsheets. The materials which are commonly utilised in the manufacture of the topsheet are polymers which are either formed into non woven fibrous layers or apertured formed films. The incorporation of such topsheets, particularly the formed films, is highly desirable in terms of fluid acquisition, reduction in rewet and masking ability. However, due to their synthetic nature these films also significantly contribute to the stickiness problem in absorbent articles. Moreover, this problem of undesirable skin feel is further exacerbated by improvements in body fit of the absorbent article.

The use of breathable backsheets in absorbent articles is another known means of improving comfort. However, it has been observed that the incorporation of breathable backsheets in absorbent articles having polymeric topsheets and/or improved body fit does not satisfactorily solve the problem of the undesirable skin feel of the products. Furthermore, increasing the breathability of such backsheets, by increasing the size of the apertures and/or the increasing the open area of the backsheet has not proven effective in addressing the problem and in some instances thereby results in an unacceptable increase of wet through onto the user's garments.

Thus, there exists a need to provide an absorbent article providing improved comfort and body fit which has reduced product to skin stickiness whilst delivering improved skin feel.

It has now been found that this problem may be addressed by the inclusion of a breathable backsheet in combination with a polymeric topsheet wherein there is a fluid contact angle gradient between the topsheet and the core. Such a gradient is achieved by the utilisation of a low surface energy material such as silicone and chlorofluorocarbons or a low surface energy treatment alone.

It is believed that the use of such low surface energy materials, increases the hydrophobicity of the topsheet of the absorbent article thereby reducing the topsheet stickiness, in spite of close product to body contact. Furthermore, an unexpected advantage of the present invention are the improved skin feel benefits which are associated with the incorporation of the breathable backsheet, which are not observed in the absence of the topsheet low surface energy treatment.

The use of surface energy gradients in absorbent articles has been described in the art such as pending US application number 08/442 935 which discloses fluid transport webs which exhibit surface energy gradients.

Summary of the Invention

The first aspect of the invention relates to a disposable absorbent article comprising a liquid pervious topsheet, an absorbent core and a breathable backsheet. The core is intermediate said topsheet and said backsheet. The backsheet, core and topsheet each comprise at least one layer, and each of said layers having a wearer facing surface and a garment facing surface and each of said surfaces having a fluid contact angle. The absorbent article has a top portion extending from and including the wearer facing surface of the core to and including the wearer facing surface of the topsheet. The garment facing surface of at least one of said layers in said top portion, has a fluid contact angle greater than the fluid contact angle of the wearer facing surface of an adjacent layer. The backsheet comprises at least one layer comprising a polymeric film having apertures having an average diameter of from 500 micrometers to 5 micrometers.

A second aspect of the invention relates to the situation wherein the wearer facing surface of at least one of said layers in the top portion has a fluid contact angle greater than the fluid contact angle of the garment facing surface of the same layer.

Detailed Description of the Invention

The present invention relates to absorbent disposable articles such as sanitary napkins, baby diapers, incontinence products and panty liners. Typically such products comprise a liquid pervious topsheet, a backsheet and an absorbent core intermediate the topsheet and the backsheet. The topsheet, backsheet and core each have a wearer facing surface and a garment facing surface. The garment facing surface of the topsheet and the wearer facing surface of the backsheet are joined to one another at the periphery of said absorbent article.

Backsheet

The absorbent articles according to the present invention comprise as an essential component a breathable backsheet. The backsheet primarily prevents the extrudes absorbed and contained in the absorbent structure from wetting articles that contact the absorbent product such as underpants, pants, pyjamas and undergarments. In addition however, the breathable backsheet of the present invention permits the transfer of both vapour and air through it and thus allows the circulation of air into and out of the backsheet.

According to the present invention the breathable backsheet comprises at least one gas permeable layer. Suitable gas permeable layers include 2 dimensional, planar micro and macro-porous films, macroscopically expanded films and formed apertured films. According to the present invention the apertures in said layer may be of any configuration, but are preferably spherical or oblong and may also be of varying dimensions. Typically, the apertures have an average diameter of from 5 micrometers to 500 micrometers. 2 dimensional planar porous films for use herein may have apertures having diameters from 200 micrometers to 5 micrometers. 2 dimensional planar microporous layers have apertures having average diameters of from 150 micrometers to 5 micrometers, preferably from 120 micrometers to 10 micrometers, most preferably from 90 micrometers to 15 micrometers. 2 dimensional planar macro porous layers have apertures having an average diameter of from 90 micrometers to 200 micrometers. Macroscopically expanded film layers and formed apertured layers have apertures having an average diameter of from 100-micrometers to 500 micrometers and may preferably have an open area of typically more than 5%, preferably from 10% to 35% of the total backsheet surface area. The apertures preferably are evenly distributed across the entire surface of the layer, however layers having only certain regions of the surface having apertures are also envisioned.

Suitable 2 dimensional planar layers of the backsheet may be made of any material known in the art, but are preferably manufactured from commonly available polymeric materials. Suitable materials are for example Goretex (TM) or Sympatex (TM) type materials well known in the art for their application in so-called breathable clothing. Other suitable materials include XMP-1001 of Minnesota Mining and Manufacturing Company, St. Paul, Minnesota, USA. As used herein the term 2 dimensional planar layer refers to layers having a depth of less than 1.5mm, preferably less than 1mm, more preferably less than 0.5mm, wherein the apertures have an average uniform diameter along their length and which do not protrude out of the plane of the layer. The apertured materials for use as a backsheet in the present invention may be produced using any of the methods known in the art such as described in EPO 293 482 and the references therein. The apertured materials may also be produced by utilising an electrical spark or discharge process. In addition the dimensions of the apertures produced by any of these methods may be increased by applying a force across the plane of the backsheet layer (i.e. stretching the layer).

Suitable apertured formed films include films which have discrete apertures which extend beyond the horizontal plane of the garment facing surface of the layer towards the core thereby forming protuberances. The protuberances have orifices located at their terminating end. Preferably said protuberances are of a funnel shape, similar to those described in US 3, 929,135. The apertures located within the plane and the orifices located at the terminating end of protuberance themselves maybe circular or non circular provided the cross sectional dimension or area of the orifice at the termination of the protuberance is smaller than the cross sectional dimension or area of the aperture located within the garment facing surface of the layer. Preferably said apertured preformed films are uni directional such that they have at least substantially, if not complete one directional fluid transport towards the core. Suitable macroscopically expanded films for use herein include films as described in for example in US 4 637 819 and US 4 591 523.

According to the present invention the backsheet may comprise in addition to said gas permeable layer additional backsheet layers. Said additional layers are located adjacent and are above or below said gas permeable layer. The additional layers may be of any material, such that they do not reduce the gas permeability of the backsheet. Preferably the second later of the backsheet is a fibrous fabric layer.

The backsheet typically extends across the whole of the absorbent structure and can extend into and form part of or all sideflaps, side wrapping elements or wings.

Absorbent core

According to the present invention, the absorbent core can include the following components: (a) an optional primary fluid distribution layer preferably together with a secondary optional fluid distribution layer; (b) a fluid storage layer;

(c) an optional fibrous ("dusting") layer underlying the storage layer; and (d) other optional components. According to the present invention the absorbent core may have any thickness depending on the end use envisioned. In a preferred embodiment of the present invention wherein the absorbent article is a sanitary napkin or a panty liner, the core may have a thickness of from 15mm to 1mm, preferably from 10mm to 1mm, most preferably from 7mm to 1mm.

a Primary/Secondary Fluid Distribution Layer

One optional component of the absorbent core according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilised. The fluid distribution layers can be comprised of any material typical for such distribution layers.

b Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials usually referred to as "hydrogel", "superabsorbent", "hydrocolloid" materials in combination with suitable carriers.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for use herein will most often comprise a substantially water-insoluble, slightly cross-linked, partially neutralised, polymeric gelling material. This material forms a hydrogel upon contact with water. Such polymer materials can be prepared from polymerizable, unsaturated, acid-containing monomers which are well known in the art.

Suitable carriers include materials which are conventionally utilised in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins and panty liners.

An embodiment of the absorbent structure made according to the present invention comprises a double layer tissue laminate formed by folding the tissue onto itself. These layers can be joined to each other for example by adhesive or by mechanical interlocking or by hydrogen bridge bonds. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

c Optional Fibrous ("Dusting") Layer

An optional component for inclusion in the absorbent core according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

d Other Optional Components of the absorbent structure

The absorbent core according to the present invention can include other optional components normally present in absorbent webs. For example, a reinforcing scrim can be positioned within the respective layers, or between the respective layers, of the absorbent core. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

Another component which can be included in the absorbent core according to the invention and preferably is provided close to or as part off the primary or secondary fluid distribution layer are odor control agents. Active carbon coated with or in addition to other odor control agents, in particular suitable zeolite or clay materials, are optionally incorporated in the absorbent structure. These components can be incorporated in any desired form but often are included as discrete particles.

The topsheet

The topsheet may comprise a single layer or a multiplicity of layers. In a preferred embodiment the topsheet comprises a first layer which provides the user facing surface of the topsheet and a second layer between the first layer and the absorbent structure/core.

The topsheet as a whole and hence each layer individually needs to be compliant, soft feeling, and non-irritating to the wearer's skin. It also can have elastic characteristics allowing it to be stretched in one or two directions. According to the present invention the topsheet may be formed from any of the materials available for this purpose and known in the art, such as non woven fabrics, films or combinations of both. In a preferred embodiment of the present invention at least one of the layers of the topsheet comprises a hydrophobic, liquid permeable apertured polymeric film. Preferably, the upper layer is provided by a film material having apertures which are provided to facilitate liquid transport from the wearer facing surface towards the absorbent structure, as detailed for example in US 3 929 135, US 4 151 240, US 4 319 868, US 4 324 426, US 4 343 314, US 4 591 523 and US 4 780 352. Alternatively, the topsheet layer may be a laminate, comprising an apertured film intermediate two fibrous layers.

The topsheet typically extends across the whole of the absorbent structure and can extend into and form part of or all of the preferred sideflaps, side wrapping elements or wings.

Fluid contact angle

According to the present invention the absorbent article has a top portion extending from and including the wearer facing surface of the core to and including the wearer facing surface of the topsheet.

According to the first aspect of the present invention any layer in said top portion has a wearer facing surface and a garment facing surface and each of said surfaces has a fluid contact angle, wherein the garment facing surface of at least one of said layers in said top portion has a fluid contact angle greater than the fluid contact angle of the wearer facing surface of an adjacent layer.

According to the second aspect of present invention any layer in said top portion has a wearer facing surface and a garment facing surface and each of said surfaces of said layers has a fluid contact angle wherein the wearer facing surface of at least one of said layers in said top portion has a fluid contact angle greater than the fluid contact angle of the garment facing surface of said same layer.

In principle the contact angle gradient may be present in said top portion between any surface (wearer facing or garment facing) of any layer therein. Thus, the fluid contact angle gradient may be present across the wearer and garment facing surface of the same layer or between the garment facing surface of at least one layer in said lower portion and an adjacent surface of an adjacent layer, i.e. between the wearer and the garment facing surface of the first layer of the topsheet, between the garment facing surface of the first layer and the wearer facing surface of the second layer of the topsheet, between the wearer and the garment facing surface of the second layer of the topsheet or between any subsequent topsheet layer. In addition, it is also foreseen that combinations of these layers each exhibiting a specific contact angle relation be utilised, thereby producing a continuous gradient in contact angles in said top portion.

Preferably the difference in fluid contact angle between two adjacent surfaces in said top portion providing a surface energy gradient should be at least 10º, preferably at least 20º and the surface having the lower surface energy should have a fluid contact angle of at least 90º, preferably at least 100º, more preferably at least 110º, most preferably at least 120º.

The principles underlying the use of surface energy gradients are outlined in pending applications US 08/268404, US 08/326571 and US 08/442935 all of which are incorporated herein by reference.

According to the present invention the contact angle of a layer may be increased by rendering that surface more hydrophobic. To manufacture a topsheet according to the present invention, a sheet of polyethylene is extruded onto a drum where it is vacuum formed into an apertured formed film and then, if desired, subjected to a corona discharge treatment generally in accordance with the teachings of U.S. Pat. Nos. 4,351,784 issued to Thomas et al. on Sept. 28, 1982; 4,456,570 issued to Thomas et al. on Jun. 26, 1984; and 4,535,020 issued to Thomas et al. on Aug. 13, 1985, the disclosures of each of these patents being incorporated herein by reference. A surface treatment having a relatively lower surface energy is then applied to the wearer facing surface of the apertured formed film and is preferably cured. A suitable surface treatment is a silicone release coating from Dow Corning of Midland, Michigan available as Syl-Off 7677 to which a crosslinker available as Syl-Off 7048 is added in proportions by weight of 100 parts to 10 parts, respectively. Another suitable surface treatment is a coating of a UV curable silicone comprising a blend of two silicones commercially available from General Electric Company, Silicone Products Division, of Waterford, NY, under the designations UV 9300 and UV 9380C-D1, in proportions by weight of 100 parts to 2.5 parts, respectively. When such a silicone blend is utilized on a formed film, coating application levels of at least 0.25g, preferably 0.5 to 8.0 grams silicone per square meter of surface area have performed satisfactorily, although other coating levels may prove suitable for certain applications depending upon the nature of the topsheet and the characteristics of the fluid, etc.

Other suitable treatment materials include, but are not limited to, fluorinated materials such as fluoropolymers (e.g., polytetrafluoroethylene (PTFE), commercially available under the trade name TEFLON") and chlorofluoropolymers. Other materials which may prove suitable for reduced surface energy include hydrocarbons such as petrolatum, latexes, paraffins, and the like, although silicone materials are presently preferred for use in the absorbent article context for their biocompatibility properties. As used herein, the term "biocompatible" is used to refer to materials having a low level of specific adsorption for, or in other words a low affinity for, bio-species or biological materials such as glucoproteins, blood platelets, and the like. As such, these materials tend to resist deposition of biological matter to a greater extent than other materials under in-use conditions. This property enables them to better retain their surface energy properties as needed for subsequent fluid handling situations. In the absence of biocompatibility, the deposition of such biological material tends to increase the roughness or non-uniformity of the surface, leading to increased drag force or resistance to fluid movement. Consequently, biocompatibility corresponds to reduced drag force or resistance to fluid movement, and hence faster access of fluid to the surface energy gradient and capillary structure. Maintenance of substantially the same surface energy also maintains the original surface energy differential for subsequent or enduring fluid depositions.

Biocompatibility, however, is not synonymous with low surface energy. Some materials, such as polyurethane, exhibit biocompatibility to some degree but also exhibit a comparatively high surface energy. Presently preferred materials such as silicone and fluorinated materials advantageously exhibit both low surface energy and biocompatibility.

Another preferred method for converting a ribbon of polyethylene film into an apertured formed film is by applying a high pressure fluid jet comprised of water or the like against one surface of the film, preferably while applying a vacuum adjacent the opposite surface of the film. Such methods are described in greater detail in commonly assigned U.S. Pat Nos. 4,609,518 issued to Curro et al. on Sept. 2, 1986; 4,629,643 issued to Curro et al. on Dec. 16, 1986; 4,637,819 issued to Ouellette et al. on Jan. 20, 1987; 4,681,793 issued to Linman et al. on July 21, 1987; 4,695,422 issued to Curro et al. on Sept. 22, 1987; 4,778,644 issued to Curro et al. on Oct. 18, 1988; 4,839,216 issued to Curro et al. on June 13, 1989; and 4,846,821 issued to Lyons et al. on July 11, 1989, the disclosures of each of said patents being incorporated herein by reference. The apertured formed film may, if desired, be subjected to a corona discharge treatment. A silicone release coating, may then be applied or printed onto the first surface of the apertured formed film and is preferably cured. The surface energy of the silicone-treated surface is less than the surface energy of the untreated surface of the topsheet.

Alternatively, the layer exhibiting the lower surface energy, e.g. the apertured polymeric topsheet or the laminate topsheet layer may have the low surface energy material incorporated within said layer during manufacture such that the layer is rendered hydrophobic during manufacture. In the case of laminated topsheets at least one of the fibrous layers is either manufactured from fibres having been treated with a low surface energy material or the fibrous layer is treated prior to the formation of the laminate itself This layer may then have a low surface energy material applied to its surface. Typically, said layer comprises at least 5% by total weight of said layer of a low surface energy material.

According to the present invention the absorbent article may find utility in sanitary napkins, panty liners, adult incontinence products and baby diapers. In particular sanitary napkins and panty liners are particularly susceptible to the present invention. Hence in addition to the components described herein, the absorbent article may comprise elastic, fastening devices and the like depending on the intended use of the article.

<u>Reference example:</u>

Examples representative of the present invention are detailed below and contact angles given in Table 1. Each test sample was prepared under identical conditions in all regards except for the specified material modification or addition as detailed. For the test samples sanitary napkins produced under the name of "Always Ultra Normal" available form Procter and Gamble GmbH, Schwalbach/Germany were manufactured according to normal manufacturing procedures.

Example 1:

In example 1 an apertured vacuum-formed-film topsheet composed of low density (LDPE) Polyethylene {supplied by Tredegar Film Products B.V. Holland under the manufacturing code X-103 2025} is chosen. The contact angle of the wearer facing surface (Ws) is treated with a basis weight of about 2 gm$^2$ thermally cured silicone. The silicone is manufactured by DOW Corning USA {sold under the trade name SYL-OFF 7048 Crosslinker/SYL-OFF 7677 Release coater (mix ratio 10% : 90%). The topsheet is combined with an absorbent core and a dual layer breathable backsheet construction. One layer of the backsheet is positioned in contact with the absorbent core is composed of a unidirectional (one way) conical apertured film (CPT) made of Low Density PE {supplied by Tredegar Film Products B.V. Holland under the manufacturing code X-1522}. The second layer of the backsheet forming the garment facing surface of the absorbent article is a nonwoven laminate {14MB/14SB manufactured by Corovin GmbH in Germany under the trade name MD 2005}. The nonwoven laminate is composed of 14 gm$^2$ spunbond and 14 gm$^2$ meltblown.

Example 2:

Is an identical structure to that of example 1 except the contact angle of the wearer facing surface (Ws) and the garment facing surface (Gs) of the topsheet is treated with a combined basis weight of about 4 gm$^2$ thermally cured silicone. The silicone is manufactured by DOW Corning USA {sold under the trade name SYL-OFF 7048 Crosslinker/SYL-OFF 7677 Release coater (mix ratio 10% : 90%). The topsheet is combined with a dual layer breathable backsheet construction as described in example 1.

Example 3:

Is an identical structure to that of example 1 except that the apertured vacuum-formed-film topsheet composed of low density (LDPE) Polyethylene topsheet{supplied by Tredegar Film Products B.V. Holland under the manufacturing code X-103 2025} is replaced by a perforated laminated nonwoven topsheet {supplied by Pantex s.r.l Italy under the manufacturing code Pantex-HO}. The wearer facing surface (ws) of the topsheet is treated with a basis weight of about 5 gsm thermally cured silicone. The silicone is manufactured by DOW Corning USA {sold under the trade name SYL-OFF 7048 Crosslinker/SYL-OFF 7677 Release coater (mix ratio 10% : 90%).

Example 4:

Is an identical structure to that of example 1 except that the breathable backsheet construction is a microapertured film {supplied by Exxon Chemical Company under the manufacturing code Exxaire XBF-102W}.

Contact Angle Determination

The contact angle test is a standard test to evaluate the nature of the interaction between a solid surface and a liquid droplet. The contact angle a droplet forms on a surface is a reflection of several interactions. The nature of the liquid, its surface tension, the nature of the solid and surface aberrations, in addition to the nature of the liquid-solid interaction. Generally speaking, a droplet on a rough surface typically exhibits a higher contact angle than a droplet on a smooth surface of the same chemical composition. If a droplet of water exhibits a contact angle greater than 90 degrees the surface is considered "hydrophobic" to the liquid. If the contact angle is less than 90 degrees then the surface is deemed "hydrophilic".

Basic Principle of the Methods:

The contact angle a liquid makes on a surface can be measured by a variety of techniques from both optical analysis of a droplet on a surface to more sophisticated techniques. The technique used herein to measure contact angle is the "Wilhelmy Plate Technique". The principle of this technique is to suspend a sample of the solid over a water vessel. The sample is then slowly lowered to a defined depth into the liquid water and then removed. The retarding force exerted by the water on the material sample on contact (zero immersion depth) is measured and the cosine of the contact angle is then determined from the equation:

$$F = ST.\ P.\ Cos\phi\ /\ g$$

Where

F    = Sample force at zero immersion depth as determined by the balance (mg)

P     = Perimeter of sample at the interface (cm)
ST    = Surface Tension (dynes cm)
Cos$\phi$  = Cosine of contact angle
g     = Acceleration due to gravity (at measuring location)

The equipment used to measure the contact angle is an Automated "Dynamic Contact Angle Analyser (model DCA-322)" manufactured by Cahn Instruments, Inc. Cerritos CA 90701-2275 USA. For each material assessed (see Table) a sample (24 mm x 30 mm) is prepared and attached to a glass slide as specified in the equipment manual. Care is taken to ensure the material sample is not touched in order to minimise contamination of the material surface. Each material is measured 5 times to ensure accuracy of measurements and to minimise impact of manufacturing variability or surface irregularities.

Surface contact angles of liquid/solid materials and surfaces (following surface tension reduction treatment) of each of the examples detailed above were measured. Additionally a comparison was also made to other materials commonly available.

| E.g | Surface material | Surface | Contact Angle Untreated | Contact Angle Treated |
|---|---|---|---|---|
| 1 | LDPE Film Code X-103 2025 Tredegar Film Products B.V. Holland | Ws | 102 | 121 |
| 2 | LDPE Film Code X-103 2025 Tredegar Film Products B.V. Holland | Ws & Gs | 102 | 144 |
| 3 | Pantex-Ho Pantex s.r.l., Pistoia Italy | Ws | 109 | 141 |
| 4 | LDPE Film Code X-103 2025 Tredegar Film Products B.V. Holland | Ws | 102 | 121 |
| Ws = wearer surface: Gs = garment surface | | | | |

The test solution utilised in this test is distilled water with a high hydrophilicity and high surface tension. This leads to contact angles that are higher than those typically found or expected to be found with menstrual fluids or urine type discharges. As such the absolute contact results detailed in the table need to be viewed with caution. A contact angle greater than 90 degrees with water does not imply that the material pores will exert a negative capillary force on menstrual type discharges. However, an increase in the contact angle will work towards lowering the extent/efficiency of liquid transport (either capillary or extrusion based) through the material in question.

**Claims**

1.  A disposable absorbent article comprising a liquid pervious topsheet, an absorbent core and a breathable backsheet, said core being intermediate said topsheet and said backsheet, said backsheet, core and topsheet each comprising at least one layer, and each of said layers having a wearer facing surface and a garment facing surface and each of said surfaces having a fluid contact angle,

    said absorbent article having a top portion extending from and including the wearer facing surface of said core to and including the wearer facing surface of said topsheet,

    wherein the garment facing surface of at least one of said layers in said top portion has a fluid contact angle greater than the fluid contact angle of the wearer facing surface of an adjacent layer and,

    wherein said backsheet comprises at least one layer comprising a polymeric film having apertures having an average diameter of from 500 micrometers to 5 micrometers.

2.  A disposable absorbent article comprising a liquid pervious topsheet, an absorbent core and a breathable backsheet, said core being intermediate said topsheet and said backsheet, said backsheet, core and topsheet each comprising at least one layer, and each of said layers having a wearer facing surface and a garment facing surface and each of said surfaces having a fluid contact angle,

    said absorbent article having a top portion extending from and including the wearer facing surface of said core to and including the wearer facing surface of said topsheet,

wherein the wearer facing surface of at least one of said layers in said top portion has a fluid contact angle greater than the fluid contact angle of the garment facing surface of said same layer and

wherein said backsheet comprises at least one layer comprising polymeric film having apertures having an average diameter of from 500 micrometers to 5 micrometers.

3. A disposable absorbent article according to any one of the preceding claims, wherein at least one of the surfaces of said layers in said top portion comprises a low surface energy material.

4. A disposable absorbent article according to claim 3, wherein said low surface energy material is selected from curable silicones, fluoropolymers, hydrocarbons or mixtures thereof.

5. A disposable absorbent article according to any one of the preceding claims, wherein either the garment facing surface or the wearer facing surface of said layer in said top portion comprises at least 0.25g of a low surface energy material per square meter of said surface.

6. A disposable absorbent article according to any of the preceding claims, wherein said backsheet comprises at least two layers a first layer comprising a polymeric apertured film and a second layer comprising a fibrous fabric layer.

7. A disposable article according to any one of the preceding claims, wherein said first layer is an apertured polymeric formed film or a 2 dimensional planar macro- or micro-porous film.

8. A disposable article according to any one of the preceding claims, wherein said first layer of the backsheet is a 2 dimensional planar film having apertures having an average diameter of from 90 micrometers to 200 micrometers.

9. A disposable article according to any one of the preceding claims, wherein said first layer of the backsheet is a 2 dimensional planar film having apertures having an average diameter of from 5 micrometers to 150 micrometers.

10. A disposable absorbent article according to any one of the preceding claims, wherein the difference in the contact angle between said surfaces according to either of claims 1 or 2 is at least 10º.

11. A disposable absorbent article according to claim 10, wherein said difference in fluid contact angle is at least 20º.

12. A disposable absorbent article according to any one of the preceding claims, wherein said fluid contact angle of said wearer facing surface of said topsheet is at least 90º.

13. A disposable absorbent article according to claim 12, wherein said fluid contact angle of said surface is at least 100º.

14. A disposable absorbent article according to claim 1 and 2, wherein said absorbent article preferably has a continuous fluid contact angle gradient in said top portion.

15. A disposable absorbent article according to any one of the preceding claims wherein said article is a sanitary napkin or a panty liner.

16. A process for the production of an absorbent article according to either of claims 1 or 2, comprising the step of applying a low surface energy material to the surface of at least said layer in said top portion.

17. A process for the production of an absorbent article according to claim 1, comprising the step of incorporating a low surface energy material within said layer.

**European Patent Office**

**EUROPEAN SEARCH REPORT**

Application Number

EP 96 83 0097

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | EP-A-0 674 892 (MCNEIL PPC INC) 4 October 1995 * column 9, line 9 - column 10, line 57; claims; figures; example * | 1-5,10, 11,14-17 | A61F13/15 |
| Y | | 6-9 | |
| Y | EP-A-0 477 802 (KIMBERLY CLARK CO) 1 April 1992 * the whole document * | 6-9 | |
| X | EP-A-0 682 930 (KIMBERLY CLARK CO) 22 November 1995 * page 3, line 14 - line 26 * * page 3, line 42 - line 48 * * page 3, line 56 - page 4, line 5 * * page 6, line 14 - line 26 * | 1-5, 15-17 | |
| D,X | WO-A-96 00548 (PROCTER & GAMBLE) 11 January 1996 * page 32, line 24 - line 36 * * page 33, line 21 - line 38 * * page 35, line 22 - line 30; claims * | 1,2 | |
| X | EP-A-0 532 005 (KIMBERLY CLARK CO) 17 March 1993 * page 7, line 14 - line 18 * * page 9, line 20 - page 10, line 5 * | 1,3,4 | TECHNICAL FIELDS SEARCHED (Int.Cl.6) A61F |
| Y | | 2 | |
| Y | EP-A-0 272 118 (PROCTER & GAMBLE) 22 June 1988 * claims * | 2 | |
| X | WO-A-94 22408 (PROCTER & GAMBLE) 13 October 1994 * page 11, line 22 - line 37 * * page 14, line 26 - line 36 * * page 18, line 24 - line 26 * | 1,3,4 | |

-/--

The present search report has been drawn up for all claims

| Place of search | Date of completion of the search | Examiner |
|---|---|---|
| THE HAGUE | 9 August 1996 | Douskas, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

## EUROPEAN SEARCH REPORT

Application Number

EP 96 83 0097

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | EP-A-0 596 532 (KIMBERLY CLARK CO) 11 May 1994 <br> * page 11, line 52 - page 12, line 14; figures * <br> --- | 1 | |
| Y | US-A-4 591 523 (THOMPSON HUGH A) 27 May 1986 <br> * claims; figures * <br> --- | 1 | |
| A | EP-A-0 235 309 (UNI CHARM CORP) 9 September 1987 <br> * page 3, line 55 - page 4, line 4; figures * <br> --- | 1 | |
| A | EP-A-0 040 084 (PROCTER & GAMBLE) 18 November 1981 <br> * page 17, line 1 - line 14; figures * <br> --- | 1 | |
| A | US-A-4 077 410 (BUTTERWORTH GEORGE A M ET AL) 7 March 1978 <br> * column 5, line 24 - line 42 * <br> ----- | 1 | **TECHNICAL FIELDS SEARCHED** (Int.Cl.6) |

The present search report has been drawn up for all claims

| Place of search | Date of completion of the search | Examiner |
|---|---|---|
| THE HAGUE | 9 August 1996 | Douskas, K |

EPO FORM 1503 03.82 (P04C01)

European Patent

Office

## CLAIMS INCURRING FEES

The present European patent application comprised at the time of filing more than ten claims.

☐ All claims fees have been paid within the prescribed time limit. The present European search report has been drawn up for all claims.

☐ Only part of the claims fees have been paid within the prescribed time limit. The present European search report has been drawn up for the first ten claims and for those claims for which claims fees have been paid,

namely claims:

☐ No claims fees have been paid within the prescribed time limit. The present European search report has been drawn up for the first ten claims.

## LACK OF UNITY OF INVENTION

The Search Division considers that the present European patent application does not comply with the requirement of unity of invention and relates to several inventions or groups of inventions, namely:

see sheet -B-

☒ As all searchable claims could be searches without effort justifying an additional fee, this Authority did not invite payment of any additional fee.

☐ All further search fees have been paid within the fixed time limit. The present European search report has been drawn up for all claims.

☐ Only part of the further search fees have been paid within the fixed time limit. The present European search report has been drawn up for those parts of the European patent application which relate to the inventions in respects of which search fees have been paid,

namely claims:

☐ None of the further search fees has been paid within the fixed time limit. The present European search report has been drawn up for those parts of the European patent application which relate to the invention first mentioned in the claims,

namely claims:

European Patent
Office

EP 96 83 0097 -B-

## LACK OF UNITY OF INVENTION

The Search Division considers that the present European patent application does not comply with the requirement of unity of invention and relates to several inventions or groups of inventions, namely:

1.  Claims 1-5,10-17    :    Absorbent article having fluid contact angle gradients between the topsheet and the absorbent core.

2.  Claims 6-9          :    Apertured backsheet for an absorbent article

EP 96 83 0097 -B-